# FASCICULE DE BREVET EUROPEEN

(11) **EP 4 496 889 B1**
(45) Date de publication et mention de la délivrance du brevet: **11.11.2026**
(21) Numéro de dépôt: 23711496.2
(22) Date de dépôt: 17.03.2023
(51) Int. Cl.: C12N 15/00, C12Q 1/6827, C12Q 1/686, C12R 1/42

(54) **PROCÉDÉ POUR DÉTECTER LE CARACTÈRE MUTAGÈNE DE COMPOSÉS CHIMIQUES**
VERFAHREN ZUR DETEKTION DES MUTAGENCHARAKTERS VON CHEMISCHEN VERBINDUNGEN
PROCESS FOR DETECTING THE MUTAGENIC CHARACTER OF CHEMICAL COMPOUNDS

(30) Priorité: 23.03.2022 FR 2202596
(43) Date de publication de la demande: 29.01.2025
(73) Titulaire: Genevolution, 78710 Rosny-sur-Seine (FR); Biomnigene, 25000 Besançon (FR)
(72) Inventeur: CARIOU, Olivier, 78710 Rosny-sur-Seine (FR); FINOT, Francis, 78117 Châteaufort (FR); ROGNON, Bénédicte, 70120 Pin (FR); BOUILLOUX, Fabrice, 25770 Chemaudin et Vaux (FR); DIDIER, Cédric, 25680 Rougemont (FR); DOUABLIN, Alexandre, 25000 Besançon (FR)
(74) Mandataire: Alatis
(86) Numéro de dépôt international: PCT/EP2023/056956
(87) Numéro de publication internationale: WO 2023/180209

(56) Documents cités:
- FR-A1- 2 775 694
- FR-A1- 3 059 429
- US-A- 5 869 258
- US-A1- 2003 211 457
- KRISTIEN MORTELMANS ET AL: "The Ames Salmonella/microsome mutagenicity assay", MUTATION RESEARCH/FUNDAMENTAL AND MOLECULAR MECHANISMS OF MUTAGENESIS, vol. 455, no. 1-2, 1 November 2000 (2000-11-01), pages 29 - 60, XP055125342, ISSN: 0027-5107, DOI: 10.1016/S0027-5107(00)00064-6
- ALI ET AL: "In vitro genotoxicity tests for polyhydroxybutyrate - A synthetic biomaterial", TOXICOLOGY IN VITRO, ELSEVIER SCIENCE, GB, vol. 22, no. 1, 19 December 2007 (2007-12-19), pages 57 - 67, XP022393667, ISSN: 0887-2333, DOI: 10.1016/J.TIV.2007.08.001

## Description

### Domaine technique de l'invention

L'invention se rapporte à l'analyse des effets des substances chimiques sur le corps humain ou animal.

L'invention a plus spécifiquement pour objet un procédé permettant de détecter l'activité mutagène de substances chimiques à des doses de l'ordre du picogramme (1 pg = 10⁻¹² g).

### Etat de la technique antérieure

En 1973, le Professeur Bruce Ames et son équipe ont mis au point un test de mutagénèse, dénommé depuis le test d'Ames, qui consiste à examiner si une substance chimique est capable d'induire des mutations spécifiques chez différentes souches de *Salmonella typbimurium* porteuses de mutations dans divers gènes gouvernant la synthèse de l'acide aminé histidine. Ces mutations préexistantes agissent comme des points chauds pour les mutagènes qui causent des dommages à l'ADN via différents mécanismes. Ces mutations rendent les souches auxotrophes à l'histidine (souches dites His⁻), c'est à dire incapables de pousser sur un milieu sans histidine. Elles réversent avec une fréquence très faible, spontanément vers His⁺, permettant à ces cellules de retrouver leur capacité à se développer sur un milieu sans histidine. Cette fréquence de réversion peut augmenter en exposant les bactéries His⁻ à des agents mutagènes. Ainsi, le test d'Ames permet de quantifier l'induction de ces mutations réverses. Lors de l'exposition de ces souches à un agent mutagène en présence ou en l'absence d'un système d'activation métabolique exogène (fraction S9 du foie), leur incapacité à pousser sur un milieu sans histidine est corrigée, en restaurant leur indépendance ou prototrophie à l'histidine. La correction peut se produire sur le site de la mutation préexistante ou à proximité. Différentes souches sont actives avec différentes classes de composés. Référence est faite à cet égard à la revue D. M. Maron et B. N. Ames publiée en 1983 (Référence 1)

La ligne directrice de l'OCDE N° 471 indique que l'essai de mutation réverse (Référence 2) doit être réalisé sur au moins cinq souches de *Salmonella typhimurium* porteuses d'une mutation dans un des gènes gouvernant la synthèse de l'acide aminé histidine, incluant obligatoirement les souches TA1535, TA1537 ou TA97a ou TA97, T98 et TA100, avec lesquelles la fiabilité et la reproductibilité de la réponse entre laboratoires ont été démontrées . La souche *Salmonella typhimurium* TA102 ou la souche *Escherichia Coli* (E.coli) WP2 uvrA (PKM101) doit aussi être utilisée pour pouvoir détecter certains mutagènes oxydants, agents de réticulation ou des hydrazines.

Des mutations ou altérations génétiques additionnelles les rendent plus sensibles à certains mutagènes chimiques particuliers. Référence est faite à cet égard à la revue de K. Mortelsmans et E. Zeiger publiée en 2000 (Référence 3).

Le protocole standard d'Ames comprend une méthode de préincubation qui consiste à exposer pendant 20 à 30 minutes dans 2 cm³ (2 ml) de tampon phosphate, 100 mm³ (100 µl) d'une culture de souche de *Salmonella typhimurium* porteuse d'une mutation dans un des gènes gouvernant la synthèse de l'acide aminé histidine, à 100 mm³ (100 µL) de substance d'essai dissoute dans de l'eau ou du DMSO en présence, soit de 500 mm³ (500 µl) d'un système d'activation métabolique (fraction S9 de foie de rat), soit d'un tampon phosphate sans système d'activation métabolique. Après une incubation supplémentaire facultative de vingt à trente minutes à 37°C, les cellules exposées sont mélangées avec 2 cm³ (2 ml) de top agar à 40-50°C contenant une faible quantité d'histidine et étalées sur des plaques de gélose solide. Après quarante-huit heures d'incubation à 37°C, les boites sont examinées, la toxicité est appréciée par l'observation du tapis bactérien et le nombre de colonies est enregistré. Chaque composé est testé en six doses couvrant une plage d'au moins trois logarithmes, chaque dose étant triplement testée. Une altération du tapis bactérien avec ou sans diminution du nombre de révertants indique une toxicité. Une augmentation du taux de réversion de plus de deux fois comparé au bruit de fond (taux de réversion observé avec le solvant), indique que le composé testé est mutagène.

Le test d'Ames traditionnel est utilisé dans le monde entier comme procédé pour détecter le potentiel mutagène de nouveaux composés. Il existe un grand nombre de variétés de test d'Ames. Un test réglementaire OCDE est réalisé directement sur les 5 souches TA98, TA100, TA97 ou TA97a ou TA1537, TA1535, TA102 ou E.Coli ; cela permet d'évaluer un large éventail de produits ayant des mécanismes d'action différents TA98/ TA1535, TA100/TA1537, TA102 ou E.coli, avec et sans la fraction S9, et répété le cas échéant pour confirmer un résultat positif. Une faible réponse sur la souche TA97 peut être confirmée avec la souche TA1537. Les souches TA102 et TA104 peuvent être utilisées si l'on soupçonne que le produit chimique peut induire des dommages oxydatifs ou être un agent de réticulation de l'ADN.

Cependant le test d'Ames tel qu'explicité au paragraphe précédent, est long dans sa mise en œuvre et il ne permet pas de détecter l'activité mutagène potentielle de composés présents à des quantités inférieures au microgramme.

Les inventeurs se sont donc attachés à mettre au point une méthode dérivée du test d'Ames qui permet de détecter l'activité mutagène de très petites quantités de composés chimiques, de l'ordre de la dizaine de picogrammes jusqu'au nanogramme.

### Exposé de l'invention

L'invention vise à remédier à tout ou partie des inconvénients mentionnés ci-dessus.

L'invention a donc pour objet un procédé pour détecter le caractère mutagène potentiel d'une substance chimique, dénommée ci-après substance chimique d'essai, mettant en œuvre les souches bactériennes de *Salmonella typhimurium* porteuses d'une mutation dans un des gènes gouvernant la synthèse de l'acide aminé histidine, dénommées respectivement souche TA97a, souche TA98, souche TA100, souche TA1535 et souche TA102, ledit procédé étant caractérisé en ce qu'il comprend, pour chacune des souches susmentionnées, dénommées ci-après souches d'essai, les étapes suivantes :
- Une étape A. de quantification par une réaction de polymérisation en chaîne, dénommée ci-après qPCR, de la proportion de gènes sauvages présents dans un premier échantillon d'une élution d'acides désoxyribonucléiques, ci-après dénommés ADNs, extraits d'une population de ladite souche d'essai, après qu'elle ait été exposée à ladite substance chimique d'essai, ledit premier échantillon ayant été en outre, préalablement à ladite qPCR, additionné d'une proportion non nulle d'un mélange d'amorces et de sondes spécifiques pour mettre en évidence les bactéries révertantes de ladite souche d'essai ;
- Une étape B. de quantification par qPCR, de la proportion de gènes mutés, présents dans un deuxième échantillon de la même élution d'ADNs que celle mise en œuvre à l'étape A., ledit deuxième échantillon ayant été en outre, préalablement à ladite qPCR, additionné d'une proportion non nulle d'un mélange d'amorces et de sondes spécifiques pour mettre en évidence les bactéries mutées de ladite souche d'essai ;
- Une étape C. mise en œuvre par ordinateur, de détermination dans l'échantillon de ladite élution d'ADNs soumis à l'étape A. et dans l'échantillon de ladite élution d'ADNs soumis à l'étape B., des quantités de gènes sauvages (Wt_{EXP}) et de gènes mutés (Mut_{EXP}) ;
- Une étape D. mise en œuvre par ordinateur, de calcul du rapport : ${R}_{EXP} = {Wt}_{EXP} / \left({Wt}_{EXP}+{Mut}_{EXP}\right)$
- Une étape E. de quantification par qPCR, de la proportion de gènes sauvages, présent dans un premier échantillon d'une élution d'ADNs extraits de la population de la même souche d'essai que celle mise en œuvre aux étapes A. et B. précédentes et qui a été uniquement exposée au solvant ayant servi à la dissolution de ladite substance chimique d'essai, ledit premier échantillon ayant été en outre, préalablement à ladite qPCR, additionné d'une proportion non nulle dudit mélange d'amorces et de sondes spécifiques mentionné à l'étape A. précédente ;
- Une étape F. de quantification par qPCR, de la proportion de gènes mutés, présents dans un deuxième échantillon de la même élution d'ADNs que celle mise en œuvre à l'étape E., ledit deuxième échantillon ayant été en outre, préalablement à ladite qPCR, additionné d'une proportion non nulle dudit mélange d'amorces et de sondes spécifiques mentionné à l'étape B. précédente ;
- Une étape G. mise en œuvre par ordinateur, de détermination dans l'échantillon de ladite élution d'ADNs soumis à l'étape E. et dans l'échantillon de ladite élution d'ADNs soumis à l'étape F., des quantités de gènes sauvages (Wt_{NEG}) et de gènes mutés (Mut_{NEG}) ; et
- Une étape H. mise en œuvre par ordinateur, de calcul du rapport : ${R}_{NEG} = {Wt}_{NEG} / \left({Wt}_{NEG}+{Mut}_{NEG}\right) .$

Le procédé tel que défini ci-dessus, permet par une méthodologie dérivant de la qPCR, ou PCR en temps réel, de quantifier le nombre de gènes sauvages et de gènes mutés dans une population de *salmonella thyphimurium* après exposition à la substance chimique d'essai et de comparer les proportions obtenues à celles observées en l'absence d'exposition à la substance d'essai. Les réactions de qPCR ciblent l'ADN total, qu'il soit issu de cellules mortes ou vivantes.

La proportion de gènes sauvages et de gènes mutés est déterminée en comparant, pour une souche donnée, les résultats de deux qPCR distinctes réalisées avec différents mélanges d'amorces et de sondes, un premier mélange spécifique du gène sauvage non muté et un second mélange spécifique du gène muté.

Les amorces oligonucléotidiques sont des oligonucléotides utilisés pour s'hybrider à une région d'un acide nucléique cible, afin de faciliter la polymérisation d'un acide nucléique complémentaire. N'importe quelle amorce peut être synthétisée par l'homme du métier. Les amorces spécifiques de chaque gène sont préparées en prenant comme référence la séquence de l'opéron histidine de *salmonella typhimurium* (ncbi accession number : JO1804.1) de manière à amplifier la région du gène qui couvre la mutation de chacune des souches TA97a, TA98, TA100, TA102 et TA1535.

Les sondes nucléotidiques sont des segments de nucléotides qui permettent de rechercher de manière spécifique un fragment d'acide nucléique que l'on désire étudier. N'importe quelle sonde peut être synthétisée par l'homme du métier. Dans le cadre de présente invention, les sondes utilisées sont des sondes froides et des séquences d'ADN.

A chaque souche correspondent deux mélanges d'amorces et de sondes spécifiques :
- Le mélange TA100WT pour amplifier spécifiquement les ADNs des souches révertantes de la souche et TA100 et le mélange TA100MUT pour amplifier spécifiquement les ADNs des souches mutées de cette souche,
- Le mélange TA98WT pour amplifier spécifiquement les ADNs des souches révertantes de la souche TA98 et le mélange TA98MUT pour amplifier spécifiquement les ADNs des souches mutées de cette souche,
- Le mélange TA102WT pour amplifier spécifiquement les ADNs des souches révertantes de la souche TA102 et le mélange TA102MUT pour amplifier spécifiquement les ADNs des souches mutées de cette souche,
- Le mélange TA97aWT pour amplifier spécifiquement les ADNs des souches révertantes de la souche TA97a et le mélange TA97aMUT pour amplifier spécifiquement les ADNs des souches mutées de cette souche, et
- Le mélange TA1535WT pour amplifier spécifiquement les ADNs des souches révertantes de la souche TA1535, et le mélange TA1535MUT pour amplifier spécifiquement les ADNs des souches mutées de cette souche.

Il y a donc deux qPCR réalisées pour chaque échantillon de souche. Ces amplifications sont effectuées avec un appareil approprié, notamment celui commercialisé par Applied Biosystems, le QuantStudio^{™} 3.0.

Dans le procédé tel que défini ci-dessus, les étapes C. et G. mises en œuvre par ordinateur, sont généralement effectuées en utilisant un logiciel associé à l'appareil de qPCR, notamment le logiciel de conception et d'analyse développé par la société QuantStudio et conçu pour analyser les données collectées à partir des systèmes de PCR (QuantStudio^{™} Design & Analysis Desktop software en langue anglaise). L'étape C. permet de déterminer le nombre de gènes sauvages (Wt_{EXP}) et de gènes mutés (Mut_{EXP}), pour chacune des cultures des souches d'essai qui a été exposée à ladite substance chimique d'essai pour en détecter l'activité mutagène potentielle. L'étape G. quant à elle, permet de déterminer le nombre de gènes sauvages (Wt_{NEG}) et de gènes mutés (Mut_{NEG}), pour chacune des cultures des souches d'essai après exposition au solvant ayant servi à la dissolution de ladite substance chimique d'essai (Témoin négatif).

Les étapes D. et H. mises en œuvre par ordinateur, sont aussi généralement effectuées en utilisant le même logiciel que celui utilisé pour les étapes C. et G. susmentionnées. Elles permettent de calculer, pour chacune des souches testées, respectivement le rapport R_{EXP} entre le nombre de gènes révertants et le nombre de gènes totaux, dans les cas où elle a été exposée à ladite substance chimique d'essai pour en détecter l'activité mutagène potentielle et le rapport R_{NEG}, dans le cas où elle a été uniquement exposée au solvant de la substance chimique dont il est souhaité détecter l'activité mutagène potentielle (Témoin négatif). Ces deux rapports sont inférieurs ou égaux à un (R_{EXP} ≤1 et R_{NEG} ≤ 1).

Grâce au procédé tel que défini ci-dessus, il est possible de comparer pour chacune des souches d'essai mentionnées, les rapports R_{EXP} et R_{NEG}. Lorsque, pour au moins une des souches d'essai, le rapport R_{EXP}/R_{NEG} est supérieur à 1, un effet mutagène de la substance chimique est détecté. Selon un mode particulier du procédé tel que défini ci-dessus, celui-ci comprend une étape I. de calcul du rapport R_{EXP}/R_{NEG.} et, plus particulièrement une étape I. mise en œuvre par ordinateur, de calcul du rapport R_{EXP}/R_{NEG.}

Selon ce mode particulier, l'étape I. mise en œuvre par ordinateur, est aussi généralement effectuée en utilisant le même logiciel que celui utilisé pour les étapes C. D., G. et H. susmentionnées.

Selon un autre mode particulier , le procédé tel que défini précédemment est caractérisé en ce qu'il comprend en outre, pour chacune des dites souches d'essai, les étapes suivantes :
- Une étape J. de quantification par qPCR, de la proportion de gènes sauvages, présents dans un premier échantillon d'une élution d'ADNs extraits de la population de la même souche d'essai que celle mise en œuvre aux étapes A., B. E. et F. précédentes, après qu'elle ait été exposée à une substance chimique de référence, ledit premier échantillon ayant été en outre, préalablement à ladite qPCR, additionné d'une proportion non nulle dudit mélange d'amorces et de sondes spécifiques mentionné à l'étape A. ;
- Une étape K. de quantification par qPCR, de la proportion de gènes mutés, présents dans un deuxième échantillon de la même élution d'ADNs que celle mise en œuvre à l'étape J., ledit deuxième échantillon ayant été en outre, préalablement à ladite qPCR, additionné d'une proportion non nulle dudit mélange d'amorces et de sondes spécifiques mentionné à l'étape B. ;
- Une étape L. mise en œuvre par ordinateur, de détermination dans l'échantillon de ladite élution d'ADNs soumis à l'étape J. et dans l'échantillon de ladite élution d'ADNs soumis à l'étape K., des quantités de gènes sauvages (Wt_{POS}) et de gènes mutés (Mut_{POS}) ;
- Une étape M. mise en œuvre par ordinateur, de calcul du rapport : ${R}_{POS} = {Wt}_{POS} / \left({Wt}_{POS}+{Mut}_{POS}\right) .$

Selon cet aspect particulier du procédé tel que défini ci-dessus, l'étape L. mise en œuvre par ordinateur, est effectuée elle-aussi en utilisant un logiciel associé à l'appareil de qPCR, par exemple, le logiciel de conception et d'analyse développé par la société QuantStudio et conçu pour analyser les données collectées à partir des systèmes de PCR (QuantStudio^{™} Design & Analysis Desktop software en langue anglaise). L'étape L. permet de déterminer le nombre de gènes sauvages (Wt_{POS}) et de gènes mutés (Mut_{POS}), pour chacune des cultures des souches d'essai qui a été exposée à ladite substance chimique de référence pour en détecter l'activité mutagène potentielle (Témoin positif). L'étape M. telle que définie ci-dessus, est elle-aussi généralement effectuée en utilisant le même logiciel que celui utilisé pour l'étape L. C'est une étape de calcul, pour chacune des souches testées, respectivement du rapport R_{POS} entre le nombre de gènes révertants et le nombre de gènes totaux, dans les cas où elle a été exposée à ladite substance chimique de référence pour en détecter l'activité mutagène potentielle (Témoin positif). Ce rapport est inférieur ou égal à un (R_{POS}≤1).

Par substance chimique de référence, on désigne une substance chimique pour laquelle il a été détectée une activité mutagène vis-à-vis d'une ou de plusieurs des souches d'essai mises en œuvre dans le procédé tel que défini ci-dessus. Il est possible de comparer pour chacune des souches d'essai mentionnées, les rapports R_{EXP} et R_{POS} et d'évaluer ainsi l'effet mutagène par rapport à celui de ladite substance chimique de référence. Ces substances chimiques de référence mises en œuvre aux étapes J. et K., sont spécifiques à une, plusieurs ou à l'ensemble des souches d'essai mises en œuvre dans le procédé tel que défini ci-dessus.

Grâce au mode particulier du procédé tel décrit ci-dessus, il est possible de comparer pour chacune des souches d'essai mentionnées, les rapports R_{EXP} et R_{POS}. Lorsque, pour une des souches d'essai, le rapport R_{EXP}/R_{POS.} est supérieur à 1, cela signifie que l'effet mutagène détecté est plus intense que celui de la substance chimique de référence.

Selon un autre mode particulier, le procédé tel que défini ci-dessus, celui-ci comprend une étape N. de calcul du rapport R_{EXP}/R_{POS}. et, plus particulièrement, une étape N. mise en œuvre par ordinateur de calcul du rapport R_{EXP}/R_{POS}.

L'étape N. telle que définie ci-dessus, est elle-aussi généralement effectuée en utilisant le même logiciel que celui utilisé pour les étapes L. et M..

L'invention a plus particulièrement pour objet le procédé tel que défini précédemment, caractérisé en ce qu'il comprend en outre, pour chacune des dites souches d'essai :
- Une étape O. de préparation des élutions d'ADNs extraits de la population de ladite souche d'essai, dont les échantillons sont mis en œuvre aux étapes A. B., E. et F. et, si désiré, aux étapes J. et K. précédentes, ladite étape O. comportant les sous-étapes suivantes :
   - Une sous-étape O1. de préparation d'une culture de ladite souche d'essai en l'introduisant dans un milieu de culture liquide supplémenté, soit en ampicilline pour chacune des dites souches TA97a, TA98, TA100, TA102 et TA1535, soit en ampicilline et en tétracycline pour ladite souches TA102, puis en y laissant croître ladite colonie dans des conditions de lumière, de température et de durée appropriées, pour obtenir au sein de ladite culture bactérienne, entre 10⁸ et 10¹⁰ cellules par centimètre cube de culture ;
   - Une sous-étape O2. de préparation d'une solution témoin négatif sans activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes d'un milieu de culture liquide, entre 2 volumes et 10 volumes d'une solution aqueuse tamponnée et entre 0,4 volume et 2 volumes du solvant utilisé pour préparer la solution initiale de ladite substance chimique d'essai ;
   - Une sous-étape O3. de préparation, d'une solution témoin négatif avec activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'un activateur métabolique et entre 0,4 volume et 2 volumes du solvant utilisé pour préparer la solution initiale de ladite substance chimique d'essai ;
   - Une sous-étape O4. de préparation d'une solution sans activateur métabolique, de ladite substance chimique d'essai, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, 2 volumes et 10 volumes d'une solution aqueuse tamponnée et entre 0,4 volume et 2 volumes d'une solution initiale comprenant entre 2,5 µg/cm³ et 300 µg/cm³ de ladite substance chimique d'essai;
   - Une sous-étape O5. de préparation d'une solution avec activateur métabolique, de ladite substance chimique d'essai, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'un activateur métabolique et entre 0,4 volume et 2 volumes d'une solution initiale comprenant entre 2,5 µg/cm³ et 300 µg/cm³ de ladite substance chimique d'essai ;
   - Optionnellement une sous-étape O6. de préparation, d'une solution témoin positif sans activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'une solution aqueuse tamponnée et entre 0,4 volume et 2 volumes d'une solution comprenant entre 2,5 µg/cm³ et 300 µg/cm³ de ladite substance chimique de référence ; et
   - Optionnellement une sous-étape O7. de préparation, d'une solution témoin positif avec activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'un activateur métabolique et entre 0,4 volume et 2 volumes d'une solution comprenant entre 2,5 µg/cm³ et 300 µg/cm³ de ladite substance chimique de référence ;
   - Une sous-étape O8. de préincubation de chacune des solutions préparées aux sous-étapes O2., O3., O4. et O5. et optionnellement aux sous-étapes O6. et O7., en les laissant au repos, à l'abri de la lumière, pendant une durée comprise entre huit et seize heures, à une température comprise entre 35°C et 45°C et plus particulièrement comprise entre 36°C et 38°C ;
   - Une sous-étape O9. d'incubation de chacune des solutions issues de la sous-étape O8. de préincubation, en mélangeant 1 volume de chacune d'entre elles avec entre 15 volumes et 25 volumes d'un milieu de culture et entre 3 volumes et 6 volumes d'une solution aqueuse tamponnée, puis en laissant chacune des dilutions résultantes au repos, à l'abri de la lumière pendant une durée comprise entre soixante et quatre-vingt-quatre heures, à une température comprise entre 35°C et 45°C et plus particulièrement comprise entre 36°C et 38°C;
   - Optionnellement une sous-étape O10. de congélation de chacune des solutions issues de la sous-étape O9. d'incubation ;
   - Optionnellement une sous-étape O11. de décongélation de chacune solutions congelées issues de la sous-étapes O10. ;
   - Une sous-étape O12. d'extraction puis d'élution des ADNs à partir de chacune des solutions, soit issues de la sous-étape O9. d'incubation, soit décongelées à l'issue de la sous-étape O11., pour obtenir les élutions mises en œuvre aux étapes A., B., E., et F. et optionnellement aux étapes J. et K, d'ADNs extraits des populations de chacune des dites souches d'essai.

Par milieu de culture liquide, on désigne de préférence un milieu liquide à température ambiante qui favorise la croissance des bactéries de type *« salmonella typhimurium* ». Un tel milieu est accessible à l'homme du métier ou il peut être aisément préparé par lui.

Dans la sous-étape O1. du procédé tel que défini ci-dessus, lesdites souches d'essai sont généralement mises à disposition sous une forme congelée dans un cryotube ou sous forme de colonies isolées.

Par conditions de lumière, de température et de durée appropriées, pour obtenir au sein de ladite culture bactérienne, entre 10⁸ et 10¹⁰ cellules par centimètre cube de culture, on signifie plus particulièrement à la sous-étape O1. du procédé tel que défini précédemment, que la culture a lieu sous agitation pendant environ 10 heures à l'abri de la lumière à une température d'environ 37°C. Selon un aspect plus particulier de la présente invention, chacune des cultures des dites souches d'essai préparées à la sous-étape O1. du procédé tel que défini ci-dessus, comportent entre 5.10⁸ et 5.10⁹ par centimètre cube de cellules de ladite souche d'essai.

Par solution aqueuse d'activateur métabolique, on entend principalement dans l'étape O du procédé tel que défini ci-dessus, une solution aqueuse comprenant 10% volumique d'une fraction surnageante d'un homogénat de foie et de rat identifiée sous le nom de fraction S-9. Une telle solution est dénommée par la suite « S9-Mix ».

La solution aqueuse tamponnée mise en œuvre dans différentes sous-étapes de l'étape O. du procédé tel que défini ci-dessus, permet d'équilibrer les proportions volumiques des solutions aqueuses préparées qui ne comprennent pas d'activateur métabolique. Comme solution aqueuse tamponnée, il y a par exemple le tampon phosphate ou le tampon acétate.

Selon un autre aspect plus particulier de la présente invention, la sous-étape O8. du procédé tel que défini ci-dessus, est effectuée à une température de37°C pendant une nuit.

Selon un autre aspect plus particulier de la présente invention, la sous-étape O9. du procédé tel que défini ci-dessus, est effectuée à une température de37°C pendant 72 heures.

Comme substances chimiques de référence utilisées pour préparer les solutions témoins positifs sans activation métabolique, il y a notamment :
L'acridine-9-amine (Numéro CAS = 90-45-9) pour ladite souche TA97a,
Le 2-nitro-*9H*-fluorène (Numéro CAS = 607-57-8) pour ladite souche TA98,
Le 4-nitroquinoline-N-oxyde (Numéro CAS = 56-57-5) pour lesdites souches TA97a, TA98, TA100, TA1535 et TA102 ,
La mitomycine C (Numéro CAS = 50-07-7) pour ladite souche TA102, et
L'azoture de sodium (Nombre CAS = 26628-22-8) pour lesdites souches TA 1535 et TA100. Comme substance chimique de référence utilisée pour préparer les solutions témoins positifs avec activation métabolique, il y a notamment l'anthracen-2-amine (Nombre CAS = 613-13-8), et le benzo[a]pyrène pour lesdites souches TA97a, TA98, TA100, TA1535 et TA102.

Comme solvants utilisés pour préparer les solutions initiales des dites substances chimiques d'essai et desdites substances chimiques de référence, il y a tout solvant qui n'interfère pas avec lesdites souches d'essai dans les conditions expérimentales mises en œuvre dans le procédé tel que défini ci-dessus. Comme exemples de solvant il y a notamment l'eau, le diméthyl sulfoxyde (DMSO), l'éthanol et l'acétonitrile.

Selon un autre mode particulier, l'invention a pour objet le procédé tel que défini précédemment, caractérisé en ce que la substance chimique de référence mise en œuvre à la sous-étape O6. est l'acridine-9-amine lorsque ladite souche d'essai est la souche TA97a, le 2-nitro-*9H*-fluorène lorsque ladite souche d'essai est la souche TA98, le 4-nitroquinoline-N-oxyde lorsque ladite souche d'essai est la souche TA100, la Mitomycine C lorsque ladite souche d'essai est la souche TA102 et l'azoture de sodium lorsque ladite souche d'essai est la souche TA1535. et en ce que la substance chimique de référence mise en œuvre à la sous-étape O7. est l'anthracen-2-amine pour toutes lesdites souches d'essai.

Selon un autre mode particulier du procédé tel que défini ci-dessus, la sous-étape O9. d'incubation est effectuée en l'absence de tout milieu de culture solide à une température inférieure à 60°C, et plus particulièrement en l'absence d'agar-agar, ou d'agar-agar modifié.

Selon un autre mode particulier du procédé tel que défini ci-dessus, ledit milieu de culture liquide mis en œuvre à chacune des sous-étapes 02 à O7. et ledit milieu de culture liquide mis en œuvre à la sous-étape O9. d'incubation, sont identiques.

Comme exemple de milieu de culture liquide exempt de de tout milieu de culture solide à une température inférieure à 60°C, et plus particulièrement en l'absence d'agar-agar, ou d'agar-agar modifié, il y a le milieu Gencar.

L'invention a plus particulièrement un procédé tel que défini précédemment, dans lequel lesdites substances chimiques d'essai sont analysées à au moins cinq concentrations différentes.

Lorsque les sous-étapes O9. et 012. sont réalisées dans des lieux différents, il est nécessaire d'effectuer la sous-étape O10. de congélation des incubats obtenus à l'issue de la sous-étape O9. puis de transférer les incubats congelés du lieu de mise en œuvre de la sous-étape O9. vers le lieu de mise en œuvre de la sous-étape O12., puis d'effectuer la sous-étape O11. de décongélation des incubats congelés, préalablement à la mise en œuvre de la sous-étape O12..

Lorsque les sous-étapes O9. et 012. sont réalisées sur un même lieu et/ou qu'un transfert de lieu est inutile, il est généralement inutile d'effectuer les sous-étapes O10. et O11. La sous-étape O12. est alors effectuée à partir de chacun des incubats directement obtenus à l'issue de la sous-étape O9..

La mise en œuvre de la sous-étape O12. du procédé tel que défini précédemment est généralement réalisée en utilisant une trousse du commerce, par exemple la trousse dénommée « Mag-Bind^{™} Bacterial DNA 96 kit» commercialisé par la société OMEGA Bioteck. Cette sous- étape inclut si nécessaire ou si désiré, une centrifugation des échantillons afin d'en éliminer les surnageants suivi d'un lavage du culot au PBS, qui sera lui-même éliminé par centrifugation. Pour contrôler la réalité et/ou l'efficacité de l'extraction des ADNs, il est utile d'effectuer une mesure spectrophotomé-trique de l'échantillon avant l'utilisation de la trousse puis une seconde mesure spectrophotomé-trique après élution des ADNs obtenus pour en déterminer la qualité et la concentration finale. Comme exemple de spectrophotomètre que l'on peut utiliser pour effecteur ces mesures, il y a notamment le nanophotomètre NP80, commercialisé par la société Implen, qui permet de quantifier les ADNs dans des échantillons liquides de volume autour de 0,5 mm³ (0,5 µl).

L'invention a aussi pour objet un procédé tel que défini ci-dessus, caractérisé en ce que la souche bactérienne d'*Escherichia coli* porteuse d'une mutation dans un gène gouvernant la synthèse de l'acide aminé tryptophane dénommée souche E.coli WP2 uvrA pKM101 est mise en œuvre, soit en remplacement ladite souche TA102, soit en complément de l'ensemble des dites souches d'essai listées précédemment.

Selon cet aspect de la présente invention, les étapes A. et E. du procédé mises en œuvre avec ladite souche E.coli WP2 uvrA pKM101, sont effectuées avec un mélange d'amorces et de sondes spécifiques pour mettre en évidence les bactéries révertantes de cette souche et les étapes B. et F. avec un mélange d'amorces et de sondes spécifiques pour mettre en évidence les bactéries mutées de cette souche. De plus, lorsqu'elle est mise en œuvre, la sous-étape O1. de préparation d'une culture de ladite souche E.coli WP2 uvrA pKM101 est effectuée en l'introduisant dans un milieu de culture liquide supplémenté, en ampicilline et en tétracycline, puis en y laissant croître ladite colonie dans des conditions de lumière, de température et de durée appropriées, pour obtenir au sein de ladite culture bactérienne, entre 10⁸ et 10¹⁰ cellules par centimètre cube de culture.

Comme substances chimiques de référence utilisées pour préparer les solutions témoins positifs lorsque ladite souche mise en œuvre est la souche E.coli WP2 uvrA pKM101, il y a notamment le 4-nitroquinoline-N-oxyde en l'absence d'activateur métabolique et l'anthracen-2-amine ou le benzo[a]pyrène en présence d'activateur métabolique.

Le procédé tel que défini précédemment, peut être avantageusement utilisé pour détecter un éventuel effet mutagène d'une substance chimique. Il est particulièrement utile pour contrôler l'innocuité des composés chimiques couramment mis en œuvre dans les produits du commerce.

C'est pourquoi, selon un dernier aspect, l'invention a pour objet le procédé tel que défini précédemment, dans lequel ladite substance chimique d'essai, dont il est souhaité détecter le caractère mutagène potentiel est un composant de produits de nettoyage, de produits cosmétiques, de produits pharmaceutiques ou de produits industriels comme les laques, les peintures ou les additifs utilisés dans tout genre d'industrie, ledit composant en étant un des excipients ou un des principes actifs.

Le procédé tel que décrit ci-dessus permet ainsi de détecter l'activité mutagène et le caractère toxique de substances chimiques présentes en de très faibles quantités dans des mélanges commerciaux de l'ordre du nanogramme, ce que ne permet pas le test d'Ames classique.

### Exemple de mise en œuvre de l'invention

L'exemple suivant illustre l'invention sans toutefois la limiter.

### A. Choix des souches et du matériel

L'essai est réalisé sur des souches de *Salmonella typhimurium* TA97a, TA98, TA100, TA102 et TA1535 soit en présence, soit en l'absence de S9-Mix dans des plaques 96-puits pour la préincubation puis dans des tubes pour l'incubation. L'incubation est réalisée en milieu liquide sans gélose ni dérivés de gélose. Les plaques 96-puits sont identifiées par le nom de la souche, la date de l'essai, la présence ou l'absence d'activation métabolique et le nom du produit à tester. Tout le matériel utilisé pour l'essai doit être stérile.

### B. Choix de l'activateur métabolique :

L'activateur métabolique utilisé est la solution S9-Mix dont la composition est indiquée dans le tableau 1 suivant :

**Tableau 1**

| **Ingrédients** | **quantités finales par cm³ (ml)** |
|---|---|
| Tampon phosphate | 100 µmoles |
| Glucose-6-phosphate (disodium) | 5 µmoles |
| NADP (disodium) | 4 µmoles |
| Chlorure de magnésium | 8 µmoles |
| Chlorure de potassium | 33 µmoles |
| S9 (fraction surnageante d'un homogénat de foie de rat commercialisée par Molecular Toxicology Inc. (USA) | 100 mm³ (100µl) |
| Eau | Jusqu'à 1 cm³ |

### C. Préparation des cultures des souches d'essai TA97a, TA98, TA100, TA1535 et TA102

### C1. A partir d'un cryotube congelé

Le volume nécessaire de souche est introduit à raison de 2% en volume (2% v/v) de milieu Gencar (milieu de culture liquide), puis supplémenté en ampicilline à raison de 25 µg/cm³ pour les souches TA97a, TA98, TA100 et TA102 et aussi de 2 µg/cm³ de tétracycline pour la souche TA102.

### C2. A partir d'une culture isolée

Une colonie de chacune des souches isolées est introduite dans 7 cm³ (7ml) de milieu Gencar puis supplémenté en ampicilline à raison de 25 µg/cm³ pour les souches TA97a, TA98, TA100 et TA102 et aussi de 2 µg/cm³ de tétracycline pour la souche TA102. Les quantités utilisées sont recensées dans le tableau 2 suivant :

**Tableau 2**

| Souche | Volumes d'ingrédients mis en œuvre | | | |
|---|---|---|---|---|
| | | Milieu Gencar | Ampicilline (10 mg/cm³) | Tétracycline (1 mg/cm³) |
| TA98 | 140 mm³ (140 µl) (ou 1 colonie) | 7 cm³ (7 ml) | 17,5 mm³ (17,5 µl) | 0 |
| TA100 | 140 mm³ (140 µl) (ou 1 colonie) | 7 cm³ (7 ml) | 17,5 mm³ (17,5 µl) | 0 |
| TA102 | 140 mm³ (140 µl) (ou 1 colonie) | 7 cm³ (7 ml) | 17,5 mm³ (17,5 µl) | 14 mm³ (14 µl) |
| TA1535 | 140 mm³ (140 µl) (ou 1 colonie) | 7 cm³ (7 ml) | 0 | 0 |
| TA97a | 140 mm³ (140 µl) (ou 1 colonie) | 7 cm³ (7 ml) | 17,5 mm³ (17,5 µl) | 0 |

**C3.** Les solutions préparées aux paragraphes C1. et C2. précédents, sont laissées pendant une nuit au bain-marie à une température de 37°C puis conservées à 4°C dès l'arrêt du bain-marie. A l'issue de cette période, les cultures bactériennes obtenues contiennent entre 5.10⁸ et 5.10⁹ cellules par centimètre cube.

### D. Préparation des solutions témoins négatifs

Ces solutions sont préparées dans les puits de plaques de quatre-vingt-seize puits selon une distribution détaillée au paragraphe G. suivant.

### D1. Solution témoin négatif sans activateur métabolique

1,5 mm³ (1,5 µl) de chaque culture bactérienne préparée à l'étape C. précédente sont mélangés à 150 mm³ (150 µl) du milieu Gencar, 6 mm³ (6 µl) de tampon phosphate et 1,2 mm³ (1,2 µl) du solvant utilisé pour préparer la solution initiale de ladite substance chimique d'essai.

### D2. Solution témoin négatif avec activateur métabolique

1,5 mm³ (1,5 µl) de chaque culture bactérienne préparée à l'étape C. précédente sont mélangés à 150 mm³ (150 µl) du milieu Gencar, 6 mm³ (6 µl) de l'activateur métabolique S9-Mix tel que défini au paragraphe B. précédent et 1,2 mm³ (1,2 µl) du solvant utilisé pour préparer la solution de ladite substance chimique d'essai.

### E. Préparation des solutions témoins positifs

Ces solutions sont préparées dans les puits de plaques de quatre-vingt-seize puits selon une distribution détaillée au paragraphe G. suivant.

### E1. Solution témoin positif sans activateur métabolique

1,5 mm³ (1,5 µl) de chaque culture bactérienne préparée à l'étape C. précédente sont mélangés à 150 mm³ (150 µl) du milieu Gencar, 1,2 mm³ (1,2 µl) de la solution de la substance chimique de référence et 6 mm³ (6 µl) de tampon phosphate.

### E2. Solution témoin négatif avec activateur métabolique :

1,5 mm³ (1,5 µl) de chaque culture bactérienne préparée à l'étape C. précédente sont mélangés à 150 mm³ (150 µl) du milieu Gencar, 1,2 mm³ (1,2 µl) de la solution de la substance chimique de référence et 6 mm³ (6 µl) de l'activateur métabolique S9-mix tel que défini au paragraphe B. précédent.

**E3.** Substances chimiques de référence utilisées : Les solutions initiales des substances chimiques de référence utilisées en l'absence et en présence d'activateur métabolique, sont recensées dans les tableaux 3 et 4 suivants :

**Tableau 3**

| Substance chimique de référence utilisée en l'absence d'activateur métabolique | | |
|---|---|---|
| Souche d'essai | | Concentration de la solution |
| TA98 | 2-nitro fluorène | 25 µg/cm³ |
| TA100 | 4 nitro quinoline-N-oxyde | 50 µg/cm³ |
| TA102 | Mitomycine | 50 µg/cm³ |
| TA1535 | Azoture de sodium | 150 µg/cm³ |
| TA97a | 9-amino acridine | 200 µg/cm³ |

**Tableau 4**

| Substance de référence utilisée en présence d'activateur métabolique | | |
|---|---|---|
| Souche d'essai | | Concentration de la solution |
| TA98 | 2-amino anthracène | 50 µg/cm³ |
| TA100 | 2-amino anthracène | 50 µg/cm³ |
| TA102 | 2-amino anthracène | 300 µg/cm³ |
| TA1535 | 2-amino anthracène | 300 µg/cm³ |
| TA97a | 2-amino anthracène | 50 µg/cm³ |

### F. Préparation des solutions de substances chimiques dont on souhaite détecter le caractère mutagène

Ces solutions sont préparées dans les puits de plaques de quatre-vingt-seize puits selon une distribution détaillée au paragraphe G. suivant. Pour chaque substance chimique d'essai à analyser, il est préparé des solutions de cinq concentrations différentes.

### F1. Solution sans activateur métabolique

1,5 mm³ (1,5 µl) de chaque culture bactérienne préparée à l'étape C. précédente sont mélangés à 150 mm³ (150 µl) du milieu Gencar, 1,2 mm³ (1,2 µl) de la solution initiale de ladite substance chimique d'essai, et 6 mm³ (6 µl) de tampon phosphate pour maintenir le pH autour de 7,4.

**F2.** Solution avec activateur métabolique : 1,5 mm³ (1,5 µl) de chaque culture bactérienne préparée à l'étape C. précédente sont mélangés à 150 mm³ (150 µl) du milieu Gencar, 1,2 mm³ (1,2 µl) de la solution initiale de ladite substance chimique d'essai et 6 mm³ (6 µL) de l'activateur métabolique S9-Mix tel que défini au paragraphe B. précédent.

### G. Etape de préincubation des souches

**G1.** Préparation des plaques de préincubation. : On prépare, pour chaque souche d'essai, une plaque de 96 puits, comportant 12 colonnes de 8 puits. En ce qui concerne les essais sans activateur métabolique,
- 5 puits de ladite plaque de 96 puits contiennent 157,5 mm³ (157,5 µl) de la solution témoin négatif, comme préparée au paragraphe D1. précédent ;
- 5 puits de ladite plaque contiennent 158,7 mm³ de la solution témoin positif, comme préparée au paragraphe E1. précédent ;
- Pour les 5 chaque concentrations choisies, 5 puits de ladite plaque contieenent 158,7 mm³ de la solution de la substance chimique d'essai, comme préparée au paragraphe F1. ;
- Le reste des puits, soit 61 puits contiennent chacun le milieu Gencar. La même distribution est adoptée pour les plaques concernant les essais avec activation métabolique avec les différentes solutions comme préparées aux paragraphes D2., E2. et F2..

**G2.** Préincubation : Les plaques comme préparées aux paragraphes G1. et G2. précédents avec chacune des souches d'essais, sont entourées par un film (type Parafilm) et laissées incuber une nuit à 37°C sous agitation.

### H. Etape d'incubation en milieu liquide

### H1. Préparation des solutions pour incubation

Les contenus de chacun des puits après préincubation, sont versés dans des tubes contenant 3 cm³ (3ml) de milieu Gencar puis dans lesquels sont incorporés 720 mm³ (720 µl) de tampon phosphate. Chaque tube contient environ 3879 mm³ (3879 µl) de liquide, dont 3225 mm³ sont finalement répartis dans 5 puits à raison de 645 mm³ (645 µl) par puits.

### H2. Incubation

Le produit en quantité de l'ordre du picogramme au microgramme est incubé en présence ou en absence de S9 dans un milieu de culture pendant un temps maximum d'une nuit.

### I. Analyse des échantillons

### I1. Conception des amorces nécessaires à la qPCR

La technologie qPCR permet l'amplification spécifique de chaque type d'allèle. La proportion de gènes sauvages et de gènes mutés est déterminée dans le présent essai en comparant les résultats de deux qPCR distinctes effectuées avec différents types d'amorces et de sondes. Un premier mélange spécifique du gène sauvage non muté, appelé mélange Wt, et un deuxième mélange spécifique du gène muté, appelé mélange Mut. Les amorces spécifiques de chaque gène sont conçues en prenant comme référence, la séquence de l'opéron histidine de *Salmonella typhimurium* (numéro ncbi : JO1804.1) de manière à amplifier la région du gène qui couvre la mutation de chacune des souches TA100, TA98, TA1535, TA102 et TA97a.

### I2. Extraction de l'ADN de cellules de Salmonella typhimurium des tubes après incubation

Les échantillons de 645 mm³ (645 µl) résultant de l'étape H d'incubation précédente, sont si nécessaire centrifugés pour éliminer tout surnageant. On y ajoute ensuite dans chacun 200 mm³ (200µl) de tampon phosphate sous poste de sécurité microbiologique (sous PSM). Après agitation vigoureuse, la densité optique (DO) à 600 nm de chaque échantillon est mesurée au moyen d'un spectrophotomètre Implen NP80. Si nécessaire les échantillons sont de nouveau centrifugés, puis leur ADN est extrait en utilisant la trousse dénommée « Mag Bind Bacterial DNA 96 kit » commercialisé par la société OMEGA bio teck. L'ADN recueilli de chaque échantillon est ensuite élué dans 50 mm³ de tampon d'élution puis la quantité et la concentration de l'ADN obtenu est mesurée au moyen du même spectrophotomètre Implen NP80 (DO à 260, 230 et 280 nm).

Des dilutions en cascade de plasmides recombinants porteurs des fragments d'intérêt sont préalablement préparés de manière à ce que les nombres de copies (amplifiées pour chacune des souches en version mutée et en version révertante) s'échelonnent de 2,5 copies/mm³ à 2,5 10⁶ copies/mm³. 2 mm³ de chaque échantillon d'ADN sont mélangés à 2 mm³ d'eau, 5 mm³ de réactif de la trousse « Genotyping Master Mix » commercialisé par la société ThermoFisher Scientific et à 1 mm³ des mélanges spécifiques d'amorces et de sondes à chaque souche suivants :
- Les mélanges TA100WT, pour amplifier spécifiquement les ADNs des souches révertantes de la souche et TA100, et TA100MUT, pour amplifier spécifiquement les ADNs des souches mutées de cette souche ;
- Les mélanges TA98WT, pour amplifier spécifiquement les ADNs des souches révertantes de la souche TA98, et TA98MUT, pour amplifier spécifiquement les ADNs des souches mutées de cette souche ;
- Les mélanges TA102WT, pour amplifier spécifiquement les ADNs des souches révertantes de la souche TA102, et TA102MUT, pour amplifier spécifiquement les ADNs des souches mutées de cette souche ;
- Les mélanges TA97aWT, pour amplifier spécifiquement les ADNs des souches révertantes de la souche TA97a et TA97aMUT pour amplifier spécifiquement les ADNs des souches mutées de cette souche ; et
- Les mélanges TA1535WT pour amplifier spécifiquement les ADNs des souches révertantes de la souche TA1535, et TA1535MUT, pour amplifier spécifiquement les ADNs des souches mutées de cette souche.

### I3. Analyse des échantillons

Deux qPCR sont réalisées pour chaque échantillon de souche, afin de quantifier pour chaque souche, la quantité de gènes sauvages non mutés et celle de gènes mutés. Ces amplifications sont effectuées avec un QuantStudio^{™} 3.0 (Applied Biosystems), en activant l'attaque à 90°C pendant 10 minutes, en effectuant 45 cycles de PCR (95°C 15 s ; 66°C 60s) avec une rampe de montée en température de 1,6°C/s. La normalisation des résultats de qPCR est permise grâce au réactif ROX contenu dans la trousse « Genotyping Master Mix »

### I4. Analyse des résultats

Les droites de régression établies à partir des valeurs des cycles de seuil (valeurs de Ct) obtenues pour chacune des gammes de dilutions de plasmide permettent de déterminer la quantité de gènes sauvages (Wt) et mutés (Mut) dans chacune des échantillons traités et leur contrôle respectif (échantillon non traité). Ces quantités sont calculées automatiquement par le logiciel associé au QuantStudio ^{™} dénommé : « QuantStudio^{™} Design & Analysis Desktop Software ». Le rapport entre le nombre de gènes révertants (Wt)et le nombre de gènes totaux (Wt + Mut) est ensuite calculé pour chacun des échantillons. La proportion de révertants dans chacun des échantillons exposés à la substance chimique dont on souhaite détecter le caractère mutagène éventuel est alors comparée à la proportion observée dans les échantillons non traités. Un rapport supérieur à 1 suggère un effet mutagène potentiel induit par ladite substance d'essai.

### Liste des documents cités

**Référence 1.** Dorothy M. Maron and Bruce N. Ames, Revised methods for the Salmonella mutagenicity test, Mutation Research, 113 (1983) 173-215 ;
**Référence 2.** Lignes directrices de l'OCDE pour les essais de produits chimiques ; Section 4 ; Ligne directrice N° 471 ; Essai de mutation réverse sur des bactéries » OCDE, 26 juin 2000 ;
**Référence 3.** Kristien Mortelmans and Errol Zeiger, The Ames Salmonella/microsome mutagenicity assay, Mutation Research 455 (2000) 29-60.

## Revendications

1. Procédé pour détecter le caractère mutagène potentiel d'une substance chimique, dénommée ci-après substance chimique d'essai, mettant en œuvre les souches bactériennes de *Salmonella typhimurium* porteuses d'une mutation dans un des gènes gouvernant la synthèse de l'acide aminé histidine, dénommées respectivement souche TA97a, souche TA98, souche TA100, souche TA1535 et souche TA102, ledit procédé étant **caractérisé en ce qu'**il comprend, pour chacune des souches susmentionnées, dénommées ci-après souches d'essai, les étapes suivantes :
- Une étape A. de quantification par une réaction de polymérisation en chaîne, dénommée ci-après qPCR, de la proportion de gènes sauvages, présents dans un premier échantillon d'une élution d'acides désoxyribonucléiques, ci-après dénommés ADNs, extraits d'une population de ladite souche d'essai, après qu'elle ait été exposée à ladite substance chimique d'essai, ledit premier échantillon ayant été en outre, préalablement à ladite qPCR, additionné d'une proportion non nulle d'un mélange d'amorces et de sondes spécifiques pour mettre en évidence les bactéries révertantes de ladite souche d'essai ;
- Une étape B. de quantification par qPCR, de la proportion de gènes mutés, présents dans un deuxième échantillon de la même élution d'ADNs que celle mise en œuvre à l'étape A., ledit deuxième échantillon ayant été en outre, préalablement à ladite qPCR, additionné d'une proportion non nulle d'un mélange d'amorces et de sondes spécifiques pour mettre en évidence les bactéries mutées de ladite souche d'essai ;
- Une étape C. mise en œuvre par ordinateur, de détermination dans l'échantillon de ladite élution d'ADNs soumis à l'étape A. et dans l'échantillon de ladite élution d'ADNs soumis à l'étape B., des quantités de gènes sauvages (Wt_{EXP}) et de gènes mutés (Mut_{EXP}) ;
- Une étape D. mise en œuvre par ordinateur, de calcul du rapport : ${R}_{EXP} = {Wt}_{EXP} / \left({Wt}_{EXP}+{Mut}_{EXP}\right)$
- Une étape E. de quantification par qPCR, de la proportion de gènes sauvages, présents dans un premier échantillon d'une élution d'ADNs, extraits de la population de la même souche d'essai que celle mise en œuvre aux étapes A. et B. précédentes et qui a été uniquement exposée au solvant ayant servi à la dissolution de ladite substance chimique d'essai, ledit premier échantillon ayant été en outre, préalablement à ladite qPCR, additionné d'une proportion non nulle dudit mélange d'amorces et de sondes spécifiques mentionné à l'étape A. précédente ;
Une étape F. de quantification par qPCR, de la proportion de gènes mutés, présents dans un deuxième échantillon de la même élution d'ADNs que celle mise en œuvre à l'étape E., ledit deuxième échantillon ayant été en outre, préalablement à ladite qPCR, additionné d'une proportion non nulle dudit mélange d'amorces et de sondes spécifiques mentionné à l'étape B. précédente ;
- Une étape G. mise en œuvre par ordinateur, de détermination dans l'échantillon de ladite élution d'ADNs soumis à l'étape E. et dans l'échantillon de ladite élution d'ADNs soumis à l'étape F., des quantités de gènes sauvages (Wt_{NEG}) et de gènes mutés (Mut_{NEG}) ; et
- Une étape H. mise en œuvre par ordinateur, de calcul du rapport : ${R}_{NEG} = {Wt}_{NEG} / \left({Wt}_{NEG}+{Mut}_{NEG}\right)$
- suivie de la comparaison, pour chacune des dites souches d'essai, des rapports R_{EXP} et R_{NEG}, afin de détecter le caractère mutagène de ladite substance chimique lorsque le rapport R_{EXP}/R_{NEG} est supérieur à 1 pour au moins une des dites souches d'essai.

2. Procédé selon la revendication 1, **caractérisé en ce qu'**il comprend en outre une étape I. mise en œuvre par ordinateur, de calcul du rapport R_{EXP}/R_{NEG}.

3. Procédé selon l'une quelconque des revendications 1 ou 2, **caractérisé en ce qu'**il comprend en outre, pour chacune des dites souches d'essai, les étapes suivantes :
- Une étape J. de quantification par qPCR, de la proportion de gènes sauvages, présents dans un premier échantillon d'une élution d'ADNs extraits de la population de la même souche d'essai que celle mise en œuvre aux étapes A. B. E. et F. précédentes, **après** qu'elle ait été exposée à une substance chimique de référence, ledit premier échantillon ayant été en outre, préalablement à ladite qPCR, additionné d'une proportion non nulle dudit mélange d'amorces et de sondes spécifiques mentionné à l'étape A. ;
- Une étape K. de quantification par qPCR, de la proportion de gènes mutés, présents dans un deuxième échantillon de la même élution d'ADNs que celle mise en œuvre à l'étape J., ledit deuxième échantillon ayant été en outre, préalablement à ladite qPCR, additionné d'une proportion non nulle dudit mélange d'amorces et de sondes spécifiques mentionné à l'étape B. ;
- Une étape L. mise en œuvre par ordinateur, de détermination dans l'échantillon de ladite élution d'ADNs soumis à l'étape J. et dans l'échantillon de ladite élution d'ADNs soumis à l'étape K., des quantités de gènes sauvages (Wt_{POS}) et de gènes mutés (Mut_{POS}) ;
- Une étape M. de calcul du rapport : ${R}_{POS} = {Wt}_{POS} / \left({Wt}_{POS}+{Mut}_{POS}\right) .$

4. Procédé selon la revendication 3, **caractérisé en ce qu'**il comprend en outre une étape N. mise en œuvre par ordinateur, de calcul du rapport R_{EXP}/R_{POS.}.

5. Procédé selon l'une quelconque des revendications 1 à 4, **caractérisé en ce qu'**il comprend en outre, pour chacune des dites souches d'essai :
- Une étape O. de préparation des élutions d'ADNs extraits de la population de ladite souche d'essai, dont les échantillons sont mis en œuvre aux étapes A. B., E. et F. et, si désiré, aux étapes J. et K. précédentes, ladite étape O. comportant les sous-étapes suivantes :
- Une sous-étape O1. de préparation d'une culture de ladite souche d'essai en l'introduisant dans un milieu de culture liquide supplémenté, soit en ampicilline pour chacune des dites souches TA97a, TA98, TA100, TA102 et TA1535, soit en ampicilline et en tétracycline pour ladite souche TA102, puis en y laissant croître ladite colonie dans des conditions de lumière, de température et de durée appropriées, pour obtenir au sein de ladite culture bactérienne, entre 10⁸ et 10¹⁰ cellules par centimètre cube de culture ;
- Une sous-étape O2. de préparation d'une solution témoin négatif sans activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes d'un milieu de culture liquide, entre 2 volumes et 10 volumes d'une solution aqueuse tamponnée et entre 0,4 volume et 2 volumes du solvant utilisé pour préparer la solution de ladite substance chimique d'essai ;
- Une sous-étape O3. de préparation d'une solution témoin négatif avec activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'un activateur métabolique et entre 0,4 volume et 2 volumes du solvant utilisé pour préparer la solution de ladite substance chimique d'essai ;
- Une sous-étape O4. de préparation d'une solution sans activateur métabolique, de ladite substance chimique d'essai, en mélangeant entre 0,5 volume et 2,5 volumes, plus particulièrement entre 0,75 volume et 1,5 volumes de ladite culture de la souche d'essai préparée à la sous-étape O1. avec entre 100 volumes et 200 volumes du même milieu de culture liquide que celui utilisé à la sous-étape 02 précédente, entre 2 volumes et 10 volumes d'une solution aqueuse tamponnée et entre 0,4 volume et 2 volumes d'une solution initiale comprenant entre 2,5 µg/cm³ et 300 µg/cm³ de ladite substance chimique d'essai ;
- Une sous-étape O5. de préparation d'une solution avec activateur métabolique, de ladite substance chimique d'essai, en mélangeant entre 0,5 volume et 2,5 volumes, plus particulièrement entre 0,75 volume et 1,5 volumes de ladite culture de la souche d'essai préparée à la sous-étape O1., avec entre 100 et 200 volumes du même milieu de culture liquide que celui utilisé à la sous-étape 02 précédente, entre 2 volumes et 10 volumes d'un activateur métabolique et entre 0,4 volume et 2 volumes d'une solution initiale comprenant entre 2,5 µg/cm³ et 300 µg/cm³ de ladite substance chimique d'essai;
- Optionnellement une sous-étape O6. de préparation d'une solution témoin positif sans activateur métabolique, en mélangeant entre 0,5 volume et 2,5 volumes, plus particulièrement entre 0,75 volume et 1,5 volumes de ladite culture de la souche d'essai préparée à la sous-étape O1., avec entre 100 et 200 volumes du même milieu de culture liquide que celui utilisé à la sous-étape 02 précédente, entre 2 volumes et 10 volumes d'une solution aqueuse tamponnée et entre 0,4 volume et 2 volumes d'une solution comprenant entre 2,5 µg/cm³ et 300 µg/cm³ de ladite substance chimique de référence ; et
- Optionnellement une sous-étape O7. de préparation d'une solution témoin positif avec activateur métabolique, en mélangeant entre 0,5 volume et 2,5 volumes, plus particulièrement entre 0,75 volume et 1,5 volumes de ladite culture de la souche d'essai préparée à la sous-étape O1. avec entre 100 et 200 volumes du même milieu de culture liquide que celui utilisé à la sous-étape 02 précédente, entre 2 volumes et 10 volumes d'un activateur métabolique et entre 0,4 volume et 2 volumes d'une solution comprenant entre 2,5 µg/cm³ et 300 µg/cm³ de ladite substance chimique de référence ;
- Une sous-étape O8. de préincubation de chacune des solutions préparées aux sous-étapes O2., O3., O4. et O5. et optionnellement aux sous-étapes O6. et O7., en les laissant au repos, à l'abri de la lumière, pendant une durée comprise entre huit et seize heures, à une température comprise entre 35°C et 45°C et plus particulièrement comprise entre 36°C et 38°C ;
- Une sous-étape O9. d'incubation de chacune des solutions issues de la sous-étape O8. de préincubation, en mélangeant 1 volume de chacune d'entre elles avec entre 15 volumes et 25 volumes d'un milieu de culture et entre 3 volumes et 6 volumes d'une solution aqueuse tamponnée, puis en laissant chacune des dilutions résultantes au repos, à l'abri de la lumière pendant une durée comprise entre soixante et quatre-vingt-quatre heures, à une température comprise entre 35°C et 45°C et plus particulièrement comprise entre 36°C et 38°C;
- Optionnellement une sous-étape O10. de congélation de chacune des solutions issues de la sous-étape O9. d'incubation ;
- Optionnellement une sous-étape O11. de décongélation de chacune solutions congelées issues de la sous-étapes O10. ;
- Une sous-étape O12. d'extraction puis d'élution des ADNs à partir de chacune des solutions, soit issues de la sous-étape O9. d'incubation, soit décongelées à l'issue de la sous-étape O11., pour obtenir les élutions mises en œuvre aux étapes A., B., E., et F. et optionnellement aux étapes J. et K, d'ADNs extraits des populations de chacune des dites souches d'essai.

6. Procédé selon la revendication 5, **caractérisée en ce que** la substance chimique de référence mise en œuvre à la sous-étape O6. est l'acridine-9-amine lorsque ladite souche d'essai est la souche TA97a, le 2-nitro-*9H*-fluorène lorsque ladite souche d'essai est la souche TA98, le 4-nitroquinoline-N-oxyde lorsque ladite souche d'essai est la souche TA100, la Mitomycine C lorsque ladite souche d'essai est la souche TA102 et l'azoture de sodium lorsque ladite souche d'essai est la souche TA1535 et **en ce que** la substance chimique de référence mise en œuvre à la sous-étape O7. pour toutes lesdites souches d'essai est l'anthracen-2-amine.

7. Procédé selon l'une quelconque des revendications 5 ou 6, **caractérisée en ce que** la sous-étape O9. d'incubation est effectuée en l'absence de tout milieu de culture solide à température inférieure à 60°C, et plus particulièrement en l'absence d'agar-agar, ou d'agar-agar modifié.

8. Procédé selon l'une quelconque des revendications 5 à 7, **caractérisée en ce que** ledit milieu de culture liquide mis en œuvre à chacune des sous-étapes O2 à O7. et ledit milieu de culture mis en œuvre à la sous-étape O9. d'incubation, sont identiques.

9. Procédé selon l'une quelconque des revendications 1 à 8, **caractérisé en ce que** ladite substance chimique d'essai, est un composant de produits de nettoyage, de produits cosmétiques, de produits pharmaceutiques ou de produits industriels comme les laques, les peintures ou les additifs utilisés dans tout genre d'industrie, ledit composant en étant un des excipients ou un des principes actifs.

## Patentansprüche

1. Verfahren zum Nachweis des potenziellen mutagenen Potenzials eines chemischen Stoffs, im Folgenden als chemischer Teststoff bezeichnet, unter Verwendung von Bakterienstämmen von *Salmonella typhimurium*, die eine Mutation in einem der Gene tragen, die die Synthese der Aminosäure Histidin steuern, und die jeweils als Stamm TA97a, Stamm TA98, Stamm TA100, Stamm TA1535 und Stamm TA102 bezeichnet werden, wobei das Verfahren **dadurch gekennzeichnet ist, dass** es für jeden der oben genannten Stämme, im Folgenden als Teststämme bezeichnet, die folgenden Schritte umfasst:
- Einen Schritt A zur Quantifizierung des Anteils an Wildtyp-Genen mittels einer Polymerase-Kettenreaktion, im Folgenden als qPCR bezeichnet, in einer ersten Probe eines Eluat von Desoxyribonukleinsäuren, im Folgenden als DNA bezeichnet, die aus einer Population des genannten Teststamms extrahiert wurden, nachdem dieser des genannten chemischen Teststoffs ausgesetzt worden war, wobei der genannten ersten Probe vor der genannten qPCR zudem ein von Null ungleicher Anteil einer Mischung aus spezifischen Primern und Sonden zugesetzt wurde, um die revertierenden Bakterien des genannten Teststamms nachzuweisen;
- Einen Schritt B. zur Quantifizierung mittels qPCR des Anteils mutierter Gene, die in einer zweiten Probe desselben DNA-Eluats vorhanden sind wie die in Schritt A. verwendete, wobei der genannten zweiten Probe vor der genannten qPCR zudem ein von Null ungleicher Anteil einer Mischung aus spezifischen Primern und Sonden zugesetzt wurde, um die mutierten Bakterien des genannten Teststamms nachzuweisen;
- Einen computerimplementierten Schritt C zur Bestimmung der Mengen an Wildtyp-Genen (Wt_{EXP}) und mutierten Genen (Mut_{EXP}) in der Probe des in Schritt A untersuchten DNA-Eluats und in der Probe des in Schritt B untersuchten DNA-Eluats;
- Einen computerimplementierten Schritt D zur Berechnung des Verhältnisses: ${R}_{EXP} = {Wt}_{EXP} / \left({Wt}_{EXP}+{Mut}_{EXP}\right)$
- Einen Schritt E. zur Quantifizierung mittels qPCR des Anteils an Wildtyp-Genen, die in einer ersten Probe eines DNA-Eluats vorhanden sind, die aus der Population desselben Teststamms extrahiert wurde, der in den vorangegangenen Schritten A. und B. verwendet wurde und der ausschließlich dem Lösungsmittel ausgesetzt war, das zur Auflösung des genannten chemischen Teststoffs diente, wobei der ersten Probe vor der qPCR zudem ein von Null ungleicher Anteil der in Schritt A. genannten Mischung aus spezifischen Primern und Sonden zugesetzt wurde;
Einen Schritt F. zur Quantifizierung mittels qPCR des Anteils mutierter Gene, die in einer zweiten Probe desselben DNA-Eluats vorhanden sind wie die in Schritt E. verwendete, wobei der besagten zweiten Probe vor der besagten qPCR zudem ein von Null ungleicher Anteil der in dem vorangegangenen Schritt B. erwähnten Mischung aus spezifischen Primern und Sonden zugesetzt wurde;
- Einen computerimplementierten Schritt G. zur Bestimmung der Mengen an Wildtyp-Genen (Wt_{NEG}) und mutierten Genen (Mut_{NEG}) in der Probe des in Schritt E. untersuchten DNA-Eluats und in der Probe des in Schritt F. untersuchten DNA-Eluats; und
- Einen computerimplementierten Schritt H. zur Berechnung des Verhältnisses: ${R}_{NEG} = {Wt}_{NEG} / \left({Wt}_{NEG}+{Mut}_{NEG}\right)$
- gefolgt vom Vergleich der Verhältnisse R_{EXP} und R_{NEG} für jeden der genannten Teststämme, um die mutagene Wirkung des genannten chemischen Stoffs nachzuweisen, wenn das Verhältnis R_{EXP} /R_{NEG} bei mindestens einem der genannten Teststämme größer als 1 ist.

2. Verfahren nach Anspruch 1, **dadurch gekennzeichnet, dass** es ferner einen computerimplementierten Schritt I. zur Berechnung des Verhältnisses R_{EXP} /R_{NEG} umfasst.

3. Verfahren nach einem der Ansprüche 1 oder 2, **dadurch gekennzeichnet, dass** es für jeden der genannten Teststämme zusätzlich die folgenden Schritte umfasst:
- einen Schritt J. zur Quantifizierung mittels qPCR des Anteils an Wildtyp-Genen, die in einer ersten Probe eines Eluats von DNA vorhanden sind, die aus der Population desselben Teststamms extrahiert wurde, der in den vorangegangenen Schritten A., B., E. und F. verwendet wurde, nachdem diese einem chemischen Referenzstoff ausgesetzt wurde, wobei der ersten Probe vor der qPCR zudem ein von Null ungleicher Anteil der in Schritt A. genannten Mischung aus spezifischen Primern und Sonden zugesetzt wurde;
- Ein Schritt K. zur Quantifizierung mittels qPCR des Anteils mutierter Gene, die in einer zweiten Probe desselben DNA-Eluats vorhanden sind wie die in Schritt J. verwendete, wobei der zweiten Probe vor der qPCR zudem ein von Null verschiedener Anteil der in Schritt B. genannten Mischung aus spezifischen Primern und Sonden zugesetzt wurde;
- Ein computerimplementierter Schritt L. zur Bestimmung der Mengen an Wildtyp-Genen (Wt_{POS}) und mutierten Genen (Mut_{POS}) in der Probe des in Schritt J. behandelten DNA-Eluats und in der Probe des in Schritt K. behandelten DNA-Eluats;
- Ein Schritt M zur Berechnung des Verhältnisses: ${R}_{POS} = {Wt}_{POS} / \left({Wt}_{POS}+{Mut}_{POS}\right) .$

4. Verfahren nach Anspruch 3, **dadurch gekennzeichnet, dass** es ferner einen computerimplementierten Schritt N zur Berechnung des Verhältnisses R_{EXP} /R_{POS.}umfasst.

5. Verfahren nach einem der Ansprüche 1 bis 4, **dadurch gekennzeichnet, dass** es für jeden der genannten Teststämme außerdem umfasst:
- einen Schritt O zur Aufbereitung der Eluate von DNA, die aus der Population des genannten Teststamms extrahiert wurde, deren Proben in den vorangegangenen Schritten A, B, E und F sowie, falls gewünscht, in den vorangegangenen Schritten J und K verwendet werden, wobei der genannte Schritt O die folgenden Teilschritte umfasst:
- einen Teilschritt O1 zur Herstellung einer Kultur des genannten Teststamms durch Einbringen in ein flüssiges Kulturmedium, das entweder mit Ampicillin für jeden der genannten Stämme TA97a, TA98, TA100, TA102 und TA1535, entweder mit Ampicillin oder mit Ampicillin und Tetracyclin für den Stamm TA102, und anschließendes Züchten der genannten Kolonie unter geeigneten Licht-, Temperatur- und Zeitbedingungen, um in der genannten Bakterienkultur zwischen 10⁸und 10¹⁰ Zellen pro Kubikzentimeter Kultur zu erhalten;
- einen Teilschritt O2 zur Herstellung einer negativen Kontrolllösung ohne Stoffwechselaktivator, indem zwischen 0,5 Volumenteilen und 2,5 Volumenteilen, insbesondere zwischen 0,75 Volumenteilen und 1,5 Volumenteilen der in Teilschritt O1 hergestellten Kultur des Teststamms mit zwischen 100 Volumenteilen und 200 Volumenteilen eines flüssigen Kulturmediums, zwischen 2 Volumenteilen und 10 Volumenteilen einer gepufferten wässrigen Lösung und zwischen 0,4 Volumenteilen und 2 Volumenteilen des Lösungsmittels, das zur Herstellung der Lösung des genannten chemischen Teststoffs verwendet wurde;
- Einen Teilschritt O3 zur Herstellung einer Negativkontrolllösung mit Stoffwechselaktivator durch Mischen von zwischen 0,5 Volumenteilen und 2,5 Volumenteilen, insbesondere zwischen 0,75 Volumenteilen und 1,5 Volumenteilen der in Teilschritt O1 hergestellten Kultur des Teststamms mit zwischen 100 Volumenteilen und 200 Volumenteilen desselben flüssigen Nährmediums, das im vorangegangenen Teilschritt O2 verwendet wurde, zwischen 2 Volumenteilen und 10 Volumenteilen eines Stoffwechselaktivators und zwischen 0,4 Volumenteilen und 2 Volumenteilen des Lösungsmittels, das zur Herstellung der Lösung des genannten chemischen Teststoffs verwendet wurde;
- Einen Teilschritt O4 zur Herstellung einer Lösung des genannten chemischen Teststoffs ohne Stoffwechselaktivator durch Mischen von 0,5 bis 2,5 Volumenteilen, insbesondere von 0,75 bis 1,5 Volumenteilen, der in Teilschritt O1 hergestellten Kultur des Teststamms mit zwischen 100 Volumenteilen und 200 Volumenteilen desselben flüssigen Nährmediums, das im vorangegangenen Teilschritt O2 verwendet wurde, zwischen 2 Volumenteilen und 10 Volumenteilen einer gepufferten wässrigen Lösung und zwischen 0,4 Volumenteilen und 2 Volumenteilen einer Ausgangslösung, die zwischen 2,5 µg/cm³ und 300 µg/cm³ des genannten chemischen Teststoffs enthält;
- Einen Teilschritt O5 zur Herstellung einer Lösung der genannten Testchemikalie mit einem Stoffwechselaktivator durch Mischen von zwischen 0,5 Volumenteilen und 2,5 Volumenteilen, insbesondere zwischen 0,75 Volumenteilen und 1,5 Volumenteilen der in Teilschritt O1 hergestellten Kultur des Teststamms, mit zwischen 100 und 200 Volumenteilen des gleichen flüssigen Nährmediums, das im vorangegangenen Teilschritt O2 verwendet wurde, zwischen 2 Volumenteilen und 10 Volumenteilen eines Stoffwechselaktivators und zwischen 0,4 Volumenteilen und 2 Volumenteilen einer Ausgangslösung, die zwischen 2,5 µg/cm³ und 300 µg/cm³ des genannten chemischen Teststoffs enthält;
- Optional einen Teilschritt O6 zur Herstellung einer positiven Kontrolllösung ohne Stoffwechselaktivator durch Mischen von zwischen 0,5 Volumenteilen und 2,5 Volumenteilen, insbesondere zwischen 0,75 Volumenteilen und 1,5 Volumenteilen der in Teilschritt O1 hergestellten Kultur des Teststamms, mit zwischen 100 und 200 Volumenteilen desselben flüssigen Nährmediums, das im vorangegangenen Teilschritt O2 verwendet wurde, zwischen 2 Volumenteilen und 10 Volumenteilen einer gepufferten wässrigen Lösung und zwischen 0,4 Volumenteilen und 2 Volumenteilen einer Lösung, die zwischen 2,5 µg/cm³ und 300 µg/cm³ des genannten chemischen Referenzstoffs enthält; und
- Optional einen Teilschritt O7 zur Herstellung einer positiven Kontrolllösung mit Stoffwechselaktivator, indem zwischen 0,5 Volumenteilen und 2,5 Volumenteilen, insbesondere zwischen 0,75 Volumenteilen und 1,5 Volumenteilen der in Teilschritt O1 hergestellten Kultur des Teststamms mit zwischen 100 und 200 Volumenteilen desselben flüssigen Nährmediums, das im vorangegangenen Teilschritt O2 verwendet wurde, zwischen 2 Volumenteilen und 10 Volumenteilen eines Stoffwechselaktivators und zwischen 0,4 Volumenteilen und 2 Volumenteilen einer Lösung, die zwischen 2,5 µg/cm³ und 300 µg/cm³ des genannten chemischen Referenzstoffs enthält;
- Einen Teilschritt O8. zur Vorinkubation jeder der in den Teilschritten O2., O3., O4. und O5. sowie optional in den Teilschritten O6. und O7. hergestellten Lösungen, indem man sie lichtgeschützt für einen Zeitraum zwischen acht und sechzehn Stunden bei einer Temperatur zwischen 35 °C und 45 °C, insbesondere zwischen 36 °C und 38 °C, ruhen lässt;
- Einen Teilschritt O9. zur Inkubation jeder der aus dem Teilschritt O8 (Vorinkubation) stammenden Lösungen, indem jeweils 1 Volumenteil jeder dieser Lösungen mit 15 bis 25 Volumenteilen eines Nährmediums und 3 bis 6 Volumenteilen einer gepufferten wässrigen Lösung gemischt wird, anschließend lässt man jede der resultierenden Verdünnungen lichtgeschützt für einen Zeitraum zwischen sechzig und vierundachtzig Stunden bei einer Temperatur zwischen 35 °C und 45 °C, insbesondere zwischen 36 °C und 38 °C, ruhen;
- Optional einen Teilschritt O10., bei dem jede der aus dem Inkubations-Teilschritt O9. stammenden Lösungen eingefroren wird;
- Optional einen Teilschritt O11 zum Auftauen jeder der aus dem Teilschritt O10 stammenden gefrorenen Lösungen;
- Einen Teilschritt O12. zur Extraktion und anschließenden Elution der DNA aus jeder der Lösungen, die entweder aus dem Teilschritt O9. (Inkubation) stammen oder nach Abschluss des Teilschritts O11. aufgetaut wurden, um die in den Schritten A., B., E. und F. sowie optional in den Schritten J. und K. verwendeten Eluate aus den Populationen der einzelnen genannten Teststämme zu erhalten.

6. Verfahren nach Anspruch 5, **dadurch gekennzeichnet, dass** die in Teilschritt O6. verwendete chemische Referenzsubstanz Acridin-9-amin ist, wenn der Teststamm der Stamm TA97a ist, 2-Nitro-9H-fluoren, wenn der Teststamm der Stamm TA98 ist, 4-Nitrochinolin-N-oxid ist, wenn der Teststamm der Stamm TA100 ist, Mitomycin C ist, wenn der Teststamm der Stamm TA102 ist, und Natriumazid ist, wenn der Teststamm der Stamm TA1535 ist, und dadurch, dass die in Teilschritt O7 verwendete Referenzchemikalie für alle genannten Teststämme Anthracen-2-amin ist.

7. Verfahren nach einem der Ansprüche 5 oder 6, **dadurch gekennzeichnet, dass** der Inkubations-Teilschritt O9. in Abwesenheit jeglichen festen Nährmediums bei einer Temperatur unter 60 °C durchgeführt wird, insbesondere in Abwesenheit von Agar-Agar oder modifiziertem Agar-Agar.

8. Verfahren nach einem der Ansprüche 5 bis 7, **dadurch gekennzeichnet, dass** das in jedem der Teilschritte O2 bis O7 verwendete flüssige Kulturmedium und das im Inkubations-Teilschritt O9 verwendete Kulturmedium identisch sind.

9. Verfahren nach einem der Ansprüche 1 bis 8, **dadurch gekennzeichnet, dass** der chemische Teststoff ein Bestandteil von Reinigungsmitteln, Kosmetika, Arzneimitteln oder Industrieprodukten wie Lacken, Farben oder Additiven ist, die in allen Arten von Industriezweigen verwendet werden, wobei dieser Bestandteil entweder ein Hilfsstoff oder ein Wirkstoff ist.

## Claims

1. A method for detecting the potential mutagenicity of a chemical substance, hereinafter referred to as the test chemical, using bacterial strains of *Salmonella typhimurium* carrying a mutation in one of the genes governing the synthesis of the amino acid histidine, referred to respectively as strain TA97a, strain TA98, strain TA100, strain TA1535 and strain TA102, respectively, said method being **characterised in that** it comprises, for each of the aforementioned strains, hereinafter referred to as test strains, the following steps:
- A step A. of quantifying, by means of a polymerase chain reaction, hereinafter referred to as qPCR, the proportion of wild-type genes present in a first sample of a eluate of deoxyribonucleic acids, hereinafter referred to as DNAs, extracted from a population of said test strain after it has been exposed to said test chemical; said first sample having, prior to said qPCR, been further supplemented with a non-zero proportion of a mixture of specific primers and probes to detect revertant bacteria of said test strain;
- A step B. involving quantification by qPCR of the proportion of mutated genes present in a second sample of the same DNA eluate as that used in step A., said second sample having, prior to said qPCR, been further supplemented with a non-zero proportion of a mixture of specific primers and probes to detect the mutated bacteria of said test strain;
- A computer-implemented step C., comprising the determination, in the sample of said DNA eluate subjected to step A. and in the sample of said DNA eluate subjected to step B., of the quantities of wild-type genes (Wt_{EXP} ) and mutated genes (Mut_{EXP} );
- A computer-implemented step D. for calculating the ratio: ${R}_{EXP} = {Wt}_{EXP} / \left({Wt}_{EXP}+{Mut}_{EXP}\right)$
- A step E. involving quantification by qPCR of the proportion of wild-type genes present in a first sample of a DNA eluate, extracted from a population of the same test strain as that used in the preceding steps A. and B., and which has been exposed solely to the solvent used to dissolve said test chemical, said first sample having, prior to said qPCR, been further supplemented with a non-zero proportion of said mixture of specific primers and probes mentioned in the preceding step A.;
A step F. involving quantification by qPCR of the proportion of mutated genes present in a second sample from the same DNA eluate as that used in step E., said second sample having, prior to said qPCR, been further supplemented with a non-zero proportion of said mixture of specific primers and probes mentioned in the preceding step B.;
- A computer-implemented step G., comprising determining, in the sample of said DNA eluate subjected to step E. and in the sample of said DNA eluate subjected to step F., the quantities of wild-type genes (Wt_{NEG} ) and mutated genes (Mut_{NEG} ); and
- A computer-implemented step H. for calculating the ratio: ${R}_{NEG} = {Wt}_{NEG} / \left({Wt}_{NEG}+{Mut}_{NEG}\right)$
- followed by a comparison, for each of the test strains, of the ratios R_{EXP} and R_{NEG} , in order to detect the mutagenic nature of the chemical substance when the ratio R_{EXP} /R_{NEG} is greater than 1 for at least one of the test strains.

2. A method according to claim 1, **characterised in that** it further comprises a computer-implemented step I. of calculating the ratio R_{EXP}/R_{NEG}.

3. A method according to any one of claims 1 or 2, **characterised in that** it further comprises, for each of said test strains, the following steps:
- A step J. of quantifying, by qPCR, the proportion of wild-type genes present in a first sample of an eluate of DNA extracted from the population of the same test strain as that used in the preceding steps A, B, E and F, after it has been exposed to a reference chemical substance, said first sample having, prior to said qPCR, been further supplemented with a non-zero proportion of said mixture of specific primers and probes mentioned in step A.;
- A step K. involving quantification by qPCR of the proportion of mutated genes present in a second sample from the same DNA eluate as that used in step J., said second sample havingbeen further supplemented, prior to said qPCR, with a non-zero proportion of said mixture of specific primers and probes mentioned in step B.;
- A computer-implemented step L. of determining, in the sample of said DNA eluate subjected to step J. and in the sample of said DNA eluate subjected to step K., the quantities of wild-type genes (Wt_{POS} ) and mutated genes (Mut_{POS} );
- Step M: calculation of the ratio: ${R}_{POS} = {Wt}_{POS} / \left({Wt}_{POS}+{Mut}_{POS}\right) .$

4. A method according to claim 3, **characterised in that** it further comprises a computer-implemented step N. for calculating the ratio R_{EXP} /R_{POS.} .

5. A method according to any one of claims 1 to 4, **characterised in that** it further comprises, for each of said test strains:
- A step O. of preparing eluates of DNA extracted from the population of said test strain, samples of which are used in steps A., B., E. and F. and, if desired, in the preceding steps J. and K., said step O. comprising the following sub-steps:
- A sub-step O1. of preparing a culture of said test strain by introducing it into a liquid culture medium supplemented either with ampicillin for each of said strains TA97a, TA98, TA100, TA102 and TA1535, or with ampicillin and tetracycline for said strain TA102, and then allowing said colony to grow therein under appropriate conditions of light, temperature and duration, to obtain within said bacterial culture between 10⁸ and 10¹⁰ cells per cubic centimetre of culture;
- A sub-step O2. comprising the preparation of a negative control solution without a metabolic activator, by mixing between 0.5 volumes and 2.5 volumes, more specifically between 0.75 volumes and 1.5 volumes, of said culture of the test strain prepared in sub-step O1. with between 100 volumes and 200 volumes of a liquid culture medium, between 2 volumes and 10 volumes of a buffered aqueous solution and between 0.4 volumes and 2 volumes of the solvent used to prepare the solution of the test chemical;
- A sub-step O3 for preparing a negative control solution with a metabolic activator, by mixing between 0.5 volumes and 2.5 volumes, more specifically between 0.75 volumes and 1.5 volumes, of said test strain culture prepared in sub-step O1 with between 100 volumes and 200 volumes of the same liquid culture medium as that used in the preceding sub-step O2, between 2 volumes and 10 volumes of a metabolic activator, and between 0.4 volumes and 2 volumes of the solvent used to prepare the solution of said test chemical;
- A sub-step O4. involving the preparation of a solution of the test chemical without a metabolic activator, by mixing between 0.5 volumes and 2.5 volumes, more specifically between 0.75 volumes and 1.5 volumes, of the test strain culture prepared in sub-step O1. with between 100 volumes and 200 volumes of the same liquid culture medium as that used in the preceding sub-step O2, between 2 volumes and 10 volumes of a buffered aqueous solution, and between 0.4 volumes and 2 volumes of an initial solution comprising between 2.5 µg/cm³ and 300 µg/cm³ of the test chemical;
- A sub-step O5. for preparing a solution containing a metabolic activator of the test chemical, by mixing between 0.5 volumes and 2.5 volumes, more specifically between 0.75 volumes and 1.5 volumes, of the test strain culture prepared in sub-step O1., with between 100 and 200 volumes of the same liquid culture medium as that used in the preceding sub-step O2, between 2 volumes and 10 volumes of a metabolic activator, and between 0.4 volumes and 2 volumes of an initial solution comprising between 2.5 µg/cm³ and 300 µg/cm³ of the test chemical;
- Optionally, a sub-step O6 for preparing a positive control solution without a metabolic activator, by mixing between 0.5 volumes and 2.5 volumes, more particularly between 0.75 volumes and 1.5 volumes, of said culture of the test strain prepared in sub-step O1, with between 100 and 200 volumes of the same liquid culture medium as that used in the preceding sub-step O2, between 2 volumes and 10 volumes of a buffered aqueous solution, and between 0.4 volumes and 2 volumes of a solution comprising between 2.5 µg/cm³ and 300 µg/cm³ of said reference chemical; and
- Optionally, a sub-step O7 for preparing a positive control solution with a metabolic activator, by mixing between 0.5 volumes and 2.5 volumes, more particularly between 0.75 volumes and 1.5 volumes, of said culture of the test strain prepared in sub-step O1 with between 100 and 200 volumes of the same liquid culture medium as that used in the preceding sub-step O2, between 2 volumes and 10 volumes of a metabolic activator, and between 0.4 volumes and 2 volumes of a solution comprising between 2.5 µg/cm³ and 300 µg/cm³ of said reference chemical;
- A sub-step O8. involving the pre-incubation of each of the solutions prepared in sub-steps O2., O3., O4. and O5., and optionally in sub-steps O6. and O7., by leaving them to stand, protected from light, for a period of between eight and sixteen hours, at a temperature of between 35°C and 45°C and, more specifically, between 36°C and 38°C;
- A sub-step O9. involving the incubation of each of the solutions obtained from sub-step O8. (pre-incubation), by mixing 1 volume of each with between 15 and 25 volumes of a culture medium and between 3 and 6 volumes of a buffered aqueous solution, then allowing each of the resulting dilutions to stand, protected from light, for a period of between sixty and eighty-four hours, at a temperature of between 35°C and 45°C and, more specifically, between 36°C and 38°C;
- Optionally, a sub-step O10. involving the freezing of each of the solutions resulting from the incubation sub-step O9.;
- Optionally, a sub-step O11. involving the thawing of each of the frozen solutions resulting from sub-step O10.;
- A sub-step O12. involving the extraction and subsequent elution of DNA from each of the solutions, either from sub-step O9. (incubation) or thawed following sub-step O11., to obtain the eluates used in steps A., B., E., and F., and optionally in steps J. and K., of DNA extracted from the populations of each of said test strains.

6. A method according to claim 5, **characterised in that** the reference chemical used in sub-step O6 is acridine-9-amine when the test strain is strain TA97a, 2-nitro-9H-fluorene when the test strain is strain TA98, 4-nitroquinoline-N-oxide when said test strain is strain TA100, mitomycin C when said test strain is strain TA102, and sodium azide when said test strain is strain TA1535; and **in that** the reference chemical used in sub-step O7. for all said test strains is anthracene-2-amine.

7. A method according to any one of claims 5 or 6, **characterised in that** sub-step O9. of incubation is carried out in the absence of any solid culture medium at a temperature below 60°C, and more particularly in the absence of agar-agar or modified agar-agar.

8. A method according to any one of claims 5 to 7, **characterised in that** the liquid culture medium used in each of sub-steps O2 to O7 and the culture medium used in incubation sub-step O9 are identical.

9. A method according to any one of claims 1 to 8, **characterised in that** said test chemical is a component of cleaning products, cosmetics, pharmaceuticals or industrial products such as lacquers, paints or additives used in all kinds of industries, said component being one of the excipients or one of the active ingredients.
